# EUROPEAN PATENT APPLICATION

(11) **EP 4 215 105 A1**
(43) Date of publication of application: **26.07.2023**
(21) Application number: 22152872.2
(22) Date of filing: 24.01.2022
(51) Int. Cl.: A61B 5/00, A61B 6/04

(54) **AUTOMATIC PAIN SENSING CONDITIONED ON A POSE OF A PATIENT**

(71) Applicant: Koninklijke Philips N.V., 5656 AG Eindhoven (NL)
(72) Inventor: KROENKE, Sven, Eindhoven (NL); YOUNG, Stewart, Eindhoven (NL); VON BERG, Jens, Eindhoven (NL); BRUECK, Heiner Matthias, Eindhoven (NL); BYSTROV, Daniel, Eindhoven (NL); GOOßEN, André, Eindhoven (NL); HARDER, Tim Philipp, Eindhoven (NL)
(74) Representative: Philips Intellectual Property & Standards

(57) **Abstract**

The present invention relates to a system and a method for detecting pain experienced by a patient in relation to a pose of the patient. The system comprises a pose estimation unit configured to estimate the pose of the patient and a pain detection unit configured to detect pain experienced by the patient. The system further comprises a processing unit configured to determine a correlation between the detected pain experienced by the patient and the estimated pose of the patient. The determined correlation can be displayed on a display unit to a physician.

## Description

### FIELD OF THE INVENTION

The present invention relates to a system for detecting pain experienced by a patient in relation to a pose of the patient, and a method for detecting pain experienced by a patient in relation to a pose of the patient.

### BACKGROUND OF THE INVENTION

For musculoskeletal diseases and trauma patients, pain-limited mobility both constitutes an important finding for the final diagnosis and can severely impact the patient positioning for medical imaging, for example X-ray examinations. Typically, pain-limited mobility is assessed during the patient's first clinical assessment, and then rediscovered during patient preparation for medical imaging. This exposes the patient to pain and stress. Up to now, there is no measurement device dedicated to detect pain conditioned on the patient's pose.

The inventors of the present invention have thus found that it would be advantageous to have a system for detecting pain experienced by a patient in relation to a pose of the patient that objectively measures and quantifies patient's pain conditioned on a pose of the patient.

### SUMMARY OF THE INVENTION

It is an object of the present invention to provide a system for detecting pain experienced by a patient in relation to a pose of the patient that objectively measures and quantifies patient's pain conditioned on a pose of the patient.

The object of the present invention is solved by the subject matter of the independent claims, wherein further embodiments are incorporated in the dependent claims.

The described embodiments similarly pertain to the system for detecting pain experienced by a patient in relation to a pose of the patient, and the method for detecting pain experienced by a patient in relation to a pose of the patient. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first embodiment of the invention, there is provided a system for detecting pain experienced by a patient in relation to a pose of the patient. The system comprises a pose estimation unit configured to estimate the pose of the patient, and a pain detection unit configured to detect pain experienced by the patient. The system further comprises a processing unit configured to determine a correlation between the detected pain experienced by the patient and the estimated pose of the patient, and a display unit configured to display the determined correlation.

Thus, the system is configured to automatically measure the pose of the patient and signals made by the patient regarding their pain perception. The pain detection unit can be configured to detect pain experienced by the patient, wherein the pain can be in correlation with the pose of the patient. The pain can be detected in correlation to the pose of the patient, and/or in correlation with a change of the pose of the patient. The processing unit of the system can be configured to determine this correlation between the detected pain and the estimated pose of the patient. In the context of the invention, the determined correlation can be understood as at least one or a plurality of pairs of the pose of the patient and the detected pain or the respective data indicative of the pain experienced by the patient at this pose. Alternatively, the correlation can be understood as, for example, a regressional curve derived from the plurality of data pairs of pose and respective pain. These findings, in particular the correlation, can be reported via the display unit to, for example, a physician. Thus, the physician can be adviced or can use the presented information to determine how to position the patient properly for a medical imaging procedure given the mobility constraints of the patient due to the experienced pain. Thereby, the present invention can help to reduce the overall patient pain and stress, since the pain and thus the mobility constraints of the patient need to be probed only once at the beginning of the medical treatment. In addition, the physician or a radiographer can be supported in finding a good compromise between a patient positioning ideal for the medical imaging and patient's comfort with little pain. Moreover, with the present invention, a clinician can be given the possibility to base his reading of the medical image and the final diagnosis on both the image and indications from the recorded patient's pain in response to his or her pose or a change of the pose. The data including pose and corresponding pain and/or its correlation measure can be attached to the acquired medical image such that this data is available for the radiologist when reading the image. Moreover, the pose can be measured or estimated in the moment of acquisition of the medical image. Having the pain and pose data available, this allows the radiologists to judge under what intensity of pain the image was acquired. This can be important, for example, when viewing the bone constellation in the medical image and judging e.g. the distribution of joint fluid for this pose.

Thus, the present invention can solve at least a part of the following problems. The patient's pain can be objectively measured and quantified conditioned on the pose as opposed to only qualitative measurements pursuit so far in the patient's first clinical assessment. Thereby, this measurement data can be leveraged for the diagnosis as well as long-term progression monitoring, e.g. for monitoring the healing process after an implant surgery. Further, pain-limited mobility is typically assessed during the patient's first clinical assessment, and then rediscovered during patient preparation for medical imaging. Thus, with the present invention, the patient needs to undergo the painful examination only once, which reduces his overall stress and pain. In addition, proper patient positioning for medical imaging, e.g. musculoskeletal X-ray examination, is challenging in the case of pain-induced mobility constraints. Having information on the mobility constraints before the preparation helps to better plan it, to adapt to the conditions and to find a good compromise between patient's comfort and ideal positioning for a given examination type. In particular, in a setting where there is already an automatic prospective or retrospective, with respect to the radiation exposure, patient-positioning quality assessment system, the acquired data can be highly valuable in order to not give erroneous quality assessments for conditions where the positioning cannot be improved further due to a lack of mobility or presence of pain. Further, the data can be used to give advice on how to improve a sub-optimal patient positioning given the mobility constraints in order to make the best out of the current situation without the risk of overdoing and thereby exposing the patient to unnecessary pain. Thereby, the overall pain and stress for the patient can be effectively reduced.

While a patient's perception of pain can be clearly subjective, and liable to vary over time, e.g. dependent upon actual swelling, healing of connective tissues, etc., it is nevertheless advantageous to create on objective record of the experienced pain, which is then available at the time of subsequent examinations, since it enables a seamless patient experience, and can be used to document temporal changes or improvements in pain perception. Although the detected grade of pain may be subjective in general and may vary between different patients and different points in time, changes in the experienced pain-strength upon changing the pose may allow for deducing an objective conclusion. Therefore, defining a mobility constraint can be achieved, for example, by determining a steep slope in the pain signal. Moreover, while the absolute pain-measurements might not be comparable among different patients, the measurements may serve as a reference in a longitudinal series of measurements for the same patient, i.e. patient monitoring.

Thus, this invention can be broadly used, for example, in all application areas dealing with musculoskeletal diseases or traumata. It can be used as a fully isolated measurement device for recording and reading these data corresponding to pain and pose for diagnosis or progression monitoring. And in particular, it can be used in combination with medical imaging like X-ray, CT, or MR for musculoskeletal diseases and traumata. Here, this invention can be closely connected to the retrospective and prospective patient-positioning assessment and guidance. In addition, a very widespread number of applications in many areas in the domains of physiotherapy, nursing and at-home patient recovery can furthermore be envisaged.

In an embodiment of the invention, the pose estimation unit is configured to estimate the pose of the patient in a time-resolved manner, and the pain detection unit is configured to detect pain experienced by the patient in a time-resolved manner.

Both the pose estimation and pain sensing can be performed synchronously during e.g. the patient's first clinical assessment, and additionally during patient preparation for a medical imaging examination. The data acquisition can take place in a time resolved manner, thus allowing, e.g., additionally detecting pain induced by the speed of a change of the pose.

In an embodiment of the invention, the system further comprises a storage unit configured to store data indicative of the detected pain experienced by the patient, data indicative of the estimated pose of the patient and/or the correlation.

The result of the measurement can be recorded and stored for further processing like compressing the measurement data by only storing the pose ranges, e.g. knee flexion angles, for which pain is absent or present. The pain record may also be edited by the examining person, orthopaedic or practician, based on the expressions of the patient after recording. By storing the measurement data like the correlation of pain and pose, the progression of the pain-induced mobility constrains can be surveilled.

In an embodiment of the invention, the pose estimation unit is configured to estimate the pose of the patient using a convolutional neural network for estimating a position of a plurality of key points of the patient on a camera image.

The patient's pose can be detected and tracked using state-of-the-art pose estimation models using e.g. convolutional neural networks for estimating keypoints on a given camera signal and fitting a skeleton model to them. Such a skeleton model allows for also calculating pose-indicative angles, e.g. the flexion angle of the knee.

In an embodiment of the invention, the pain detection unit is configured to detect pain experienced by the patient based on a camera image and/or based on an audio signal.

The pain detection unit can be configured to analyze a camera image of at least a part of the patient in order to detect pain experienced by the patient. In addition or alternatively, an audio signal can be analyzed to detect pain experienced by the patient.

Thus, potential signals made by the patient regarding their pain perception can be automatically detected and potentially also graded w.r.t. to pain intensity using e.g. an RGB video of the patient's face or a recorded audio signal. A machine learning algorithm can be trained to e.g. recognize facial expressions corresponding to pain or sounds associated with pain. In embodiments of the present invention, natural language processing can be used in order to also analyse spoken words of the patient for pain indications.

In an embodiment of the invention, the pain detection unit is configured to detect pain experienced by the patient based on the camera image using a machine learning algorithm trained to recognize a facial expression of the patient on the camera image corresponding to pain experienced by the patient.

In addition or alternatively, the pain detection unit and/or the machine learning algorithm can be configured to recognize a facial expression of the patient. The facial expression can be indicative of pain experienced by the patient, such like grimacing.

In order to train a machine-learning algorithm which can regress the level of pain from a certain type of input data, e.g. color-images of the face, in supervised manner, a training dataset needs to be created. This can be accomplished in several ways: Patients can be asked rate the degree of pain on a scale 1 to 5 in a time-series of measurements, i.e. for each measurement instance, the input data and the corresponding pain rating are recorded. Alternatively, an expert can rate the strength of pain retrospectively for each input data sample in a time-series of measurements.

In an embodiment of the invention, the pain detection unit is configured to detect pain experienced by the patient based on the camera image using a machine learning algorithm trained to recognize a fluidity and/or speed of a motion of the patient.

In addition or alternatively, the pain detection unit and/or the machine learning algorithm can be configured to recognize a fluidity and/or speed of a motion of the patient. The fluidity and/or speed of motion of the patient can be indicative of pain experienced by the patient. For example, if the patient feels pain, his motions will be slower and less fluid. When the patient is observed via cameras, an objective measure of mobility can be derived by analysing the fluidity and overall speed of the patient motions, thereby mimicking the radiographers experience in understanding the patient's limitations.

In an embodiment of the invention, the pain detection unit is configured to detect pain experienced by the patient based on the audio signal using a machine learning algorithm trained to recognize sounds associated with pain.

In addition or alternatively, the pain detection unit and/or the machine learning algorithm can be configured to recognize sounds associated with pain. Thus, the machine learning algorithm can detect sounds made by the patient such like sighs or screams and can translate them into a degree of pain experienced by the patient.

In an embodiment of the invention, the pain detection unit is configured to detect pain experienced by the patient based on a physical and/or linguistic input of the patient.

Thus, the input of the patient to the pain detection unit can be, for example, verbal input informing the pain detection unit whether or not and how strong he experiences pain. In addition or alternatively, the patient can press a button or operate an interface in order to grade his pain. Thus, the patient can use a number code to express the grade of pain. Also a specific device like a push button or a device to measure the strength of biting can be used to record the degree of pain simultaneously with the pose.

In an embodiment of the invention, the processing unit is configured to determine a mobility constraint of the patient based on the correlation between the detected pain experienced by the patient and the estimated pose of the patient.

Thus, the system can determine, for example, the range in which the patient can move a joint without excessive pain. When flexing a joint of the patient, for example the knee, the patient can experience an increasing pain when exceeding a specific flexing angle. This specific angle can be regarded as mobility constraint which should not be exceeded during imaging, for example, in order to reduce pain and stress of the patient.

In an embodiment of the invention, the processing unit is configured to determine a positioning of the patient for a medical imaging procedure based on the mobility constraint.

Thus, the system can use the determined mobility constraint in combination with predefined requirements regarding the positioning of the patient for a medical imaging procedure in order to determine an optimal positioning of the patient during the imaging procedure. The optimal positioning can be defined by the best compromise between pain experienced by the patient and a high quality of the acquired medical images.

In an embodiment of the invention, the system is configured to compare the determined correlation with a record of an earlier correlation of the same patient.

The system can be configured to store measurement data indicative of pose and respective pain and/or the determined correlation. These stored data can be used as a record of an earlier examination, and can be compared with the measurement data and/or correlation of another examination following, for example, an intervention, a surgical procedure, or a time span of recovery and healing.

According to another aspect of the invention, there is provided a method for detecting pain experienced by a patient in relation to a pose of the patient using the system according to any of the preceding embodiments. The method comprising the steps of estimating the pose of the patient and detecting pain experienced by the patient. The method comprises further the steps of determining a correlation between the detected pain experienced by the patient and the estimated pose of the patient, and displaying the determined correlation.

Thus, according to this method, the pose of a patient is measured. As the patient might feel pain corresponding to the pose, the pain experienced by the patient in correlation to the pose is detected. The correlation between pose and respective pain can be determined. In the context of the invention, the correlation can be regarded as both pairs of measurement data of pose and pain and functional relation between pose and pain that can be derived by mathematical analysis. The functional relation can be, for example, an interpolation, a fit or a regression to the data points. The results of the measurement of pose and pain can displayed, thus reporting it to a physician.

In an embodiment of the invention, the previously described method is a computer implemented method. A system including a computer can be configured to execute the steps of the method described above. Thus, the method can be automatically performed by the system.

In an embodiment of the invention the method for detecting pain experienced by a patient in relation to a pose of the patient uses the system according to any of the preceding embodiments. The method comprising the steps of estimating, using the pose estimation unit of the system, the pose of the patient and detecting, using the pain detection unit of the system, pain experienced by the patient. The method comprises further the steps of determining, using the processing unit of the system, a correlation between the detected pain experienced by the patient and the estimated pose of the patient, and displaying the determined correlation on the display unit of the system.

Thus, the method can be performed using a system comprising a pose estimation unit, a pain detection unit, a processing unit and a display unit.

According to another aspect of the invention, there is provided a computer program element, which, when executed on a processing unit, instructs the processing unit to cause the method according to any of the preceding embodiments.

The computer program element can be performed on one or more processing units, which are instructed to cause the method for detecting pain experienced by a patient in relation to a pose of the patient.

Preferably, the program element is stored in a system for detecting pain experienced by a patient in relation to a pose of the patient and a processing unit carrying out this program element is part of said system.

The computer program element may be part of a computer program, but it can also be an entire program by itself. For example, the computer program element may be used to update an already existing computer program to get to the present invention.

The computer program element may be stored on a computer readable medium. The computer readable medium may be seen as a storage medium, such as for example, a USB stick, a CD, a DVD, a data storage device, a hard disk, or any other medium on which a program element as described above can be stored.

According to another aspect of the invention, there is provided a processing unit configured for executing the computer program element according to the preceding embodiment.

The processing unit can be distributed over one or more different devices executing the computer program element according to the invention.

Thus, the benefits provided by any of the above aspects equally apply to all of the other aspects and vice versa.

In a gist, the invention relates to a system and a method for detecting pain experienced by a patient in relation to a pose of the patient. The system comprises a pose estimation unit configured to estimate the pose of the patient and a pain detection unit configured to detect pain experienced by the patient. The system further comprises a processing unit configured to determine a correlation between the detected pain experienced by the patient and the estimated pose of the patient. The determined correlation can be displayed on a display unit to a physician.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a schematic set-up of a system for detecting pain experienced by a patient in relation to a pose of the patient according to an embodiment of the invention.
Fig. 2 shows a block diagram of a method for detecting pain experienced by a patient in relation to a pose of the patient according to another embodiment of the invention.

### DETAILED DESCRIPTION OF EMBODIMENTS

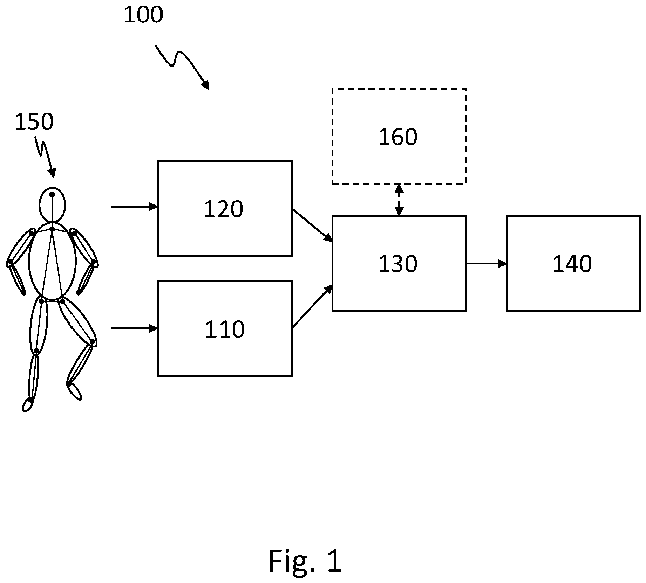

Fig. 1 shows a schematic set-up of a system 100 for detecting pain experienced by a patient 150 in relation to a pose of the patient according to an embodiment of the invention. A subject like a patient 150 can be observed by a pose estimation unit that can be a camera, for example. The camera detects the pose of the patient, who can be provided with a plurality of marks attached to keypoints like joints of the patient. Thus, the pose estimation unit 110 can detect the pose of the patient 150, which can be defined by the angle of at least one joint of the patient 150. Preferably simultaneously, a pain detection unit 120 detects pain experienced by the patient 150. In embodiments of the invention, the pain detection unit 120 can include a camera, which can be the same as the camera of the pose estimation unit, but can also be another camera. The camera of the pain detection unit 120 preferably takes images of the face of the patient 150 and the pain detection unit 120 is configured to analyse the images of the face of the patient in order to derive a measure of pain experienced by the patient. This pain is preferably the result of a pose taken by the patient 150 due to an injury or handicap of the patient 150. Thus, the pain detection unit 120 detects the pain experienced by the patient 150. The processing unit 130 can be configured to analyse the estimated pose of the patient 150 and the corresponding data indicative of the pain experienced by the patient 150 and detected by the pain detection unit 120. The processing unit 130 can be configured to analyse at which pose or position the patient experiences pain, at which angle of a joint the pain increases significantly, or which pose or angle of a joint is comfortable for the patient 150. Thus the processing unit 130 can correlate the estimated pose with the detected pain experienced by the patient 150. The correlation can be displayed on a display unit 140 to a physician or a radiographer, for example. In addition or alternatively, the correlation can be stored on a storage unit 160. In the context of the invention, the term of the correlation can be understood as any form of data indicating the relationship between pose and pain, which can be, for example, at least on data point including a pair of pose with the respective pain or a plurality of data points or a functional relationship like a regression. Therefore, the system 100 can, for example, help to determine an optimal positioning of a patient for a medical imaging procedure, where the patient 150 needs not to suffer unnecessary pain. In addition or alternatively, the system can help to provide a diagnosis or a measure of progress in healing of the patient. The stored data of consecutive measurements of the pose and corresponding pain of the patient 150 can be utilized as a record of the history and healing process of the patient, or can indicate a deterioration of the condition of the patient. Therefore, the system 100 according to the invention can be applied in many fields of treatment and diagnosis of patients.

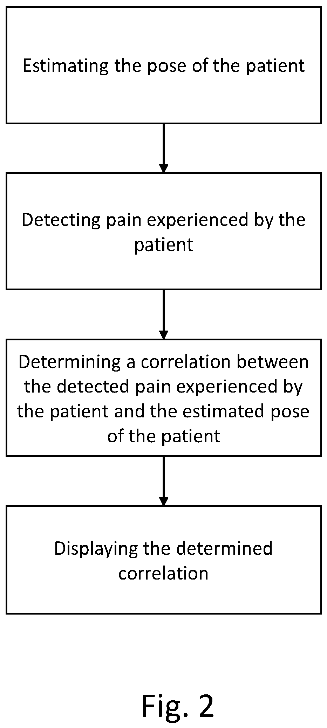

Fig. 2 shows a block diagram of a method for detecting pain experienced by a patient 150 in relation to a pose of the patient according to another embodiment of the invention. In a first step, the pose of the patient is estimated. In a second step, pain experienced by the patient is detected. The pain can correspond to the pose of the patient. In a third step, a correlation between the detected pain experienced by the patient and the estimated pose of the patient is determined. This correlation can be a plurality of datasets each comprising a pose and data indicative of the respective pain. Alternatively, the correlation can be, for example, an equation describing the pain as a function of the pose, which can be derived from an analysis of the plurality of pairs of pose and pain. In a fourth step, the determined correlation can be displayed on a display unit 140.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: system for detecting pain
- 110: pose estimation unit
- 120: pain detection unit
- 130: processing unit
- 140: display unit
- 150: patient
- 160: storage unit

## Claims

1. A system (100) for detecting pain experienced by a patient (150) in relation to a pose of the patient, the system comprising:
a pose estimation unit (110) configured to estimate the pose of the patient (150);
a pain detection unit (120) configured to detect pain experienced by the patient (150);
a processing unit (130) configured to determine a correlation between the detected pain experienced by the patient (150) and the estimated pose of the patient; and
a display unit (140) configured to display the determined correlation.

2. The system (100) according to claim 1,
wherein the pose estimation unit (110) is configured to estimate the pose of the patient in a time-resolved manner; and
wherein the pain detection unit (120) is configured to detect pain experienced by the patient in a time-resolved manner.

3. The system (100) according to any of the preceding claims,
wherein the system further comprises a storage unit (160) configured to store data indicative of the detected pain experienced by the patient (150), data indicative of the estimated pose of the patient (150) and/or the correlation.

4. The system (100) according to any of the preceding claims,
wherein the pose estimation unit (110) is configured to estimate the pose of the patient (150) using a convolutional neural network for estimating a position of a plurality of key points of the patient (150) on a camera image.

5. The system (100) according to any of the preceding claims,
wherein the pain detection unit (120) is configured to detect pain experienced by the patient (150) based on a camera image and/or based on an audio signal.

6. The system (100) according to claim 5,
wherein the pain detection unit (120) is configured to detect pain experienced by the patient (150) based on the camera image using a machine learning algorithm trained to recognize a facial expression of the patient (150) on the camera image corresponding to pain experienced by the patient.

7. The system (100) according to any of claims 5 or 6,
wherein the pain detection unit (120) is configured to detect pain experienced by the patient (150) based on the camera image using a machine learning algorithm trained to recognize a fluidity and/or speed of a motion of the patient (150).

8. The system (100) according to any of claims 5 to 7,
wherein the pain detection unit (120) is configured to detect pain experienced by the patient (150) based on the audio signal using a machine learning algorithm trained to recognize sounds associated with pain.

9. The system (100) according to any of the preceding claims,
wherein the pain detection unit (120) is configured to detect pain experienced by the patient (150) based on a physical and/or linguistic input of the patient (150).

10. The system (100) according to any of the preceding claims,
wherein the processing unit (130) is configured to determine a mobility constraint of the patient (150) based on the correlation between the detected pain experienced by the patient and the estimated pose of the patient.

11. The system (100) according to claim 10,
wherein the processing unit (130) is configured to determine a positioning of the patient (150) for a medical imaging procedure based on the mobility constraint.

12. The system (100) according to any of the preceding claims,
wherein the system (100) is configured to compare the determined correlation with a record of an earlier correlation of the same patient (150).

13. A method for detecting pain experienced by a patient (150) in relation to a pose of the patient (150), the method comprising the steps of:
estimating the pose of the patient;
detecting pain experienced by the patient;
determining a correlation between the detected pain experienced by the patient and the estimated pose of the patient; and
displaying the determined correlation.

14. A computer program element, which, when executed on a processing unit (130), instructs the processing unit to perform the method according to claim 13.

15. A processing unit (130) configured for executing the computer program element according to claim 14.
